# EUROPEAN PATENT APPLICATION

(11) **EP 4 685 807 A1**
(43) Date of publication of application: **28.01.2026**
(21) Application number: 25190840.6
(22) Date of filing: 21.07.2025
(51) Int. Cl.: G16H 15/00, A61B 5/00, G16H 50/20, G16H 50/30, G16H 30/20, G16H 80/00

(54) **PRENATAL SCREENING INFORMATION PROCESSING METHOD AND APPARATUS, COMPUTER DEVICE, STORAGE MEDIUM**

(30) Priority: 23.07.2024 CN 202410994776
(71) Applicant: Shenzhen New Industries Biomedical Engineering Co., Ltd., Shenzhen, Guangdong 518122 (CN)
(72) Inventor: TANG, Junhui, Shenzhen (CN); ZHANG, Zhi, Shenzhen (CN); HUI, Jiangchao, Shenzhen (CN); ZHU, Junjie, Shenzhen (CN); LAN, Jia, Shenzhen (CN); WANG, Xueli, Shenzhen (CN); ZHENG, Yanlin, Shenzhen (CN)
(74) Representative: Metida

(57) **Abstract**

A prenatal screening information processing method and apparatus, a computer equipment, and a storage medium are provided. When a target pregnant woman is in a multifetal pregnancy state, a plurality of first information components respectively corresponding to a plurality of fetuses are displayed on the prenatal screening information processing interface; each first information component includes an essential information unit and a risk information unit; the essential information unit is configured to display essential information of corresponding fetuses; and the risk information unit is configured to display risk information of the corresponding fetuses. By using the method, the information of the plurality of fetuses, such as the essential information and the risk information, can be synchronously displayed intensively on the prenatal screening information processing interface, making it convenient for a user to view the information uniformly.

## Description

### TECHNICAL FIELD

The present invention relates to the technical field of prenatal screening, and in particular, to a prenatal screening information processing method and apparatus, a computer device, and a storage medium.

### BACKGROUND

Prenatal screening means a screening test on pregnant women during pregnancy, and is aimed at testing whether a fetus has some common genetic diseases or chromosomal abnormalities. For example, in a narrow sense, Down's syndrome screening means testing the blood of pregnant women to test concentrations of alpha fetoprotein, chorionic gonadotropin, and free estriol in maternal serum by testing blood of a pregnant woman, and determining, based on the age, the weight, and the gestational weeks of the pregnant woman, risk coefficients of Down's syndrome and a neural tube defect in a fetus. Existing prenatal screening risk calculation software only supports a singleton pregnancy mode. In a multifetal pregnancy situation, such as in a twins pregnancy situation, on pregnant woman basis, two "different" pregnant women may be generated based on two different sample numbers. Each pregnant woman corresponds to one fetus. Therefore, when fetus information and prenatal examination results are displayed, information of fetuses of the same pregnant woman and corresponding prenatal examination results are bound to files of the different pregnant women. Consequently, the information of the fetuses of the same pregnant woman and the corresponding prenatal examination results are displayed separately and cannot be viewed together.

### SUMMARY

In view of this, it is necessary to provide a prenatal screening information processing method and apparatus, a computer device, and a computer-readable storage medium, to solve technical problems that information of multiple fetuses cannot be displayed intensively, information needs to be entered repeatedly, and the like in the prior art.

In a first aspect, the present invention provides a prenatal screening information processing method, including: displaying a prenatal screening information processing interface; and if a target pregnant woman is in a multifetal pregnancy state, displaying a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

In one embodiment, the prenatal screening information processing interface further includes a second information component; the second information component includes a basic information unit and a test information unit; and the method further includes: displaying basic information of the target pregnant woman by the basic information unit, displaying test information of the target pregnant woman by the test information unit, and displaying the essential information of the corresponding fetuses through the essential information units, where the basic information includes a multifetal pregnancy identification; the multifetal pregnancy identification is used to identify whether the target pregnant woman is in the multifetal pregnancy state; and determining the risk information of each fetus based on at least one of the basic information of the target pregnant woman, the test information of the target pregnant woman, and the basic information of each fetus.

In one embodiment, the displaying basic information of the target pregnant woman by the basic information unit, displaying test information of the target pregnant woman by the test information unit, and displaying the essential information of the corresponding fetuses through the essential information units includes: in response to a first entering operation triggered on the second information component, respectively displaying, in the basic information unit and the test information unit, the basic information and test information of the target pregnant woman that are entered based on the first entering operation; and in response to a second entering operation triggered on the first information components, displaying, in the corresponding essential information units, the essential information of each fetus entered based on the second entering operation.

In one embodiment, the displaying basic information of the target pregnant woman by the basic information unit, displaying test information of the target pregnant woman by the test information unit, and displaying the essential information of the corresponding fetuses through the essential information units further includes: obtaining, from an associated information management system, the basic information and test information of the target pregnant woman, and the essential information of each fetus of the target pregnant woman; and respectively displaying the basic information and test information of the target pregnant woman in the basic information unit and the test information unit, and displaying the essential information of the corresponding fetuses in the essential information units.

In one embodiment, the associated information management system includes a laboratory information management system; the prenatal screening information processing interface includes a data processing region; the data processing region includes a review state control; the obtaining, from an associated information management system, the basic information and test information of the target pregnant woman, and the essential information of each fetus of the target pregnant woman includes: in response to a triggering operation on the review state control, transmitting an update request to the laboratory information management system, where the update request is used to instruct the laboratory information management system to obtain unreviewed pregnant woman information from a database, and the pregnant woman information includes basic information and test information of a pregnant woman, and essential information of a corresponding fetus of the pregnant woman; receiving updated pregnant woman information transmitted by the laboratory information management system, and displaying a candidate pregnant women list in the data processing region based on the updated pregnant woman information; and determining the target pregnant woman from the candidate pregnant women list, and obtaining the basic information and test information of the target pregnant woman, and the essential information of each fetus of the target pregnant woman.

In one embodiment, the determining the risk information of each fetus based on at least one of the basic information of the target pregnant woman, the test information of the target pregnant woman, and the basic information of each fetus further includes: synchronizing fetus information of each fetus corresponding to the target pregnant woman to prenatal screening risk calculation software for storage, where the fetus information includes at least one of the essential information and the risk information.

In one embodiment, the synchronizing fetus information of each fetus corresponding to the target pregnant woman to prenatal screening risk calculation software for storage includes: determining a first fetus and at least one second fetus from a plurality of fetuses based on at least one of the essential information and the risk information; calling a synchronization interface of the prenatal screening risk calculation software, and synchronizing a first data file to a first storage position of the prenatal screening risk calculation software, where the first data file includes the basic information and test information of the target pregnant woman, and the fetus information of the first fetus; the first storage position is used to store data that is recognized and displayed by the prenatal screening risk calculation software; and synchronizing a second data file to a second storage position of the prenatal screening risk calculation software, where the second data file includes the basic information and test information of the target pregnant woman, and the fetus information of the second fetus; and the second storage position is used to store data that does not need to be displayed by the prenatal screening risk calculation software.

In one embodiment, the essential information unit includes a gestational week calculation method control; the essential information further includes a gestational week calculation result; and a mode for obtaining the gestational week calculation result includes: in response to a triggering operation on the gestational week calculation method control, determining a gestational week calculation method; displaying a corresponding gestational week calculation factor entering unit based on the gestational week calculation method; and when an entering operation on the gestational week calculation factor entering unit satisfies a preset calculation condition, displaying the gestational week calculation result, where the preset calculation condition includes a value entered by the entering operation being within a preset calculation range.

In one embodiment, the prenatal screening information processing interface further includes a report generation control; and the method further includes: in response to a triggering operation on the report generation control, generating a prenatal screening report, where prenatal screening report includes the essential information and the risk information that respectively correspond to the plurality of fetuses of the target pregnant woman.

In a second aspect, the present invention provides a prenatal screening information processing apparatus, including: an interface display module, configured to display a prenatal screening information processing interface; and an information component display module, configured to: if a target pregnant woman is in a multifetal pregnancy state, display a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

In a third aspect, the present invention further provides a computer device. The computer device includes a memory and a processor. The memory has a computer program stored therein. The processor, when executing the computer program, implements the following steps: displaying a prenatal screening information processing interface; and if a target pregnant woman is in a multifetal pregnancy state, displaying a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

In a fourth aspect, the present invention further provides a computer-readable storage medium. The computer-readable storage medium has a computer program stored therein. The computer program, when executed by a processor, implements the following steps: displaying a prenatal screening information processing interface; and if a target pregnant woman is in a multifetal pregnancy state, displaying a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

According to the prenatal screening information processing method and apparatus, the computer equipment, and the storage medium, when a target pregnant woman is in a multifetal pregnancy state, a plurality of first information components respectively corresponding to a plurality of fetuses are displayed on the prenatal screening information processing interface; each first information component includes an essential information unit and a risk information unit; the essential information unit is configured to display essential information of corresponding fetuses; and the risk information unit is configured to display risk information of the corresponding fetuses. By using the method, the information of the plurality of fetuses, such as the essential information and the risk information, can be synchronously displayed intensively on the prenatal screening information processing interface, making it convenient for a user to view the information uniformly.

### BRIEF DESCRIPTION OF THE DRAWINGS

FIG. 1 is a diagram of an application environment of a prenatal screening information processing method in some embodiments;
FIG. 2 is a flowchart of a prenatal screening information processing method in some embodiments;
FIG. 3 is a schematic diagram of displaying of multifetal information in one embodiment;
FIG. 4 is a schematic diagram of displaying of multifetal information in another embodiment;
FIG. 5 is a schematic diagram of a review state control in some embodiments;
FIG. 6 is a schematic diagram of a report template in some embodiments;
FIG. 7 is a structural block diagram of a prenatal screening information processing apparatus in some embodiments; and
FIG. 8 is a diagram of an internal structure of a computer device in some embodiments.

### DETAILED DESCRIPTION

In order to make the objectives, technical solutions, and advantages of the present invention clearer, the following is a further detailed explanation of the present invention in conjunction with the accompanying drawings and embodiments. It should be understood that the specific embodiments described herein are merely used to explain the present invention but are not intended to limit the present invention.

The terms "include," "contain," "have," and any variations thereof used in the present invention are intended to cover non-exclusive inclusions. For example, processes, methods, systems, products, or devices that contains a series of steps or units are not necessarily limited to the steps or units clearly listed, but may also include other steps or units that are not clearly listed or inherent to these processes, methods, products, or devices.

The term "plurality" used in the present invention means more than two (including two), unless otherwise specifically limited.

The terms "first", "second", and the like used in the present invention are used to distinguish similar objects by names, but these objects themselves are not limited by these terms and cannot be understood as indicating or implying relative importance or implying quantities, specific orders, or primary and secondary relations of the technical features indicated. It should be understood that these terms may be interchangeable in appropriate circumstances without departing from the scope of the present invention. For example, "first fetus" can be described as "second fetus", and similarly, "second fetus" can be described as "first fetus".

The term "and/or" used in the present invention only describes an association relationship for describing associated objects, representing that three relationships may exist. For example, A and/or B may represent three situations: A exists alone; A and B exist simultaneously; and B exists alone.

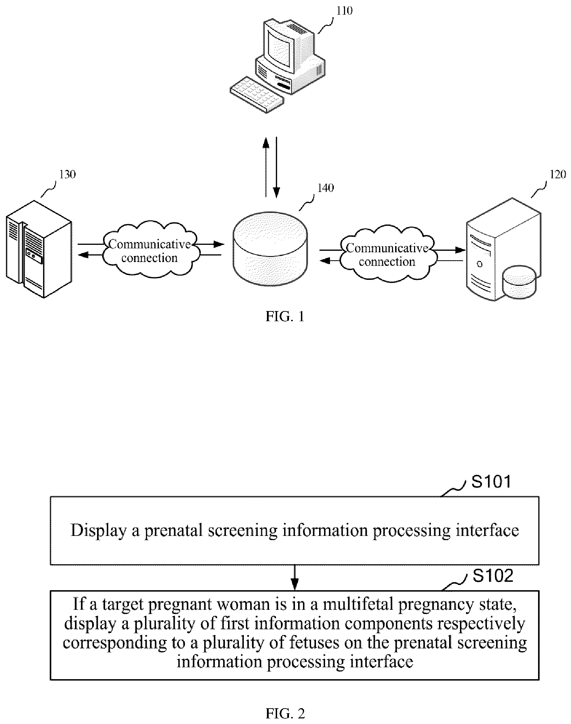

A prenatal screening information processing method provided in the embodiments of the present invention may be applied to an application environment shown in FIG. 1. A control terminal 110, a laboratory information management system 120, a test instrument 130, and a prenatal screening report system server 140 are included. The control terminal 110 is installed with a prenatal screening report system client. The control terminal 110 accesses the prenatal screening report system server 140 through the prenatal screening report system client installed on the control terminal, to call relevant functions of its server and read data in a database. The prenatal screening report system server 140 is respectively in communicative connection to the laboratory information management system 120, the test instrument 130, and the control terminal 110. Typically, the database of the prenatal screening report system server 140 can also be installed on the control terminal 110. The control terminal 110 directly calls data of the prenatal screening report system server 140 that is locally stored.

Specifically, the laboratory information management system 120 is configured to store basic information of a pregnant woman, test information of a sample of the pregnant woman, and associated fetus information such as essential information, risk information, and the like of a fetus. The prenatal screening report system client installed on the control terminal 110 provides a prenatal screening information processing interface for viewing the data in the prenatal screening report system server 140. The test instrument 130 is configured to: test the sample of the pregnant woman and directly output the test information of the sample. The test information is transmitted to the laboratory information management system 120 through the prenatal screening report system server 140. Similarly, the prenatal screening report system server 140 may directly obtain the test information or obtain the test information through the laboratory information management system 120. Further, the database of the prenatal screening report system server 140 may be used to store the basic information of the pregnant woman. The prenatal screening report system server 140 implements information data transmission and synchronization by the communicative connection to the system, the test instrument, or the terminal, or may be offline with the system or test instrument and used separately in conjunction with the prenatal screening report system client on the control terminal 110.

The control terminal 110 may include, but is not limited to, various desktop computers, laptops, tablet computers, Internet of things (IoT) devices, and smart phones. Data information storage of the laboratory information management system 120 may be implemented by using an independent server or a server cluster composed of a plurality of servers.

The present invention will be further described in detail below with reference to the accompanying drawings and embodiments. It should be noted that the following explanation uses an example in which the method provided in the present invention is applied to the control terminal in FIG. 1, but this is only an illustrative example and is not limited to only performing the method by the control terminal.

It should be understood that at least some of steps in FIG. 2 may include a plurality of sub-steps or stages which are not necessarily completed at the same time, but can be executed at different times. Execution orders of these sub-steps or stages are not necessarily sequential, but these sub-steps or stages and at least some of other steps or sub-steps or stages of other steps can be executed by turns or alternately.

FIG. 2 is a flowchart of a prenatal screening information processing method provided in an embodiment of the present invention. The method may be performed by a prenatal screening information processing apparatus. The device may be implemented by software and/or hardware and may typically be integrated into an electronic device. An integration mode may be mounting a client into a corresponding electronic device. The client may be an application program for displaying a prenatal screening information processing interface, a web client, or a sub-application that is run in an operating environment of a parent application. The electronic device may be a mobile device such as a mobile phone, a smart watch, a tablet computer, or a personal digital assistant, or may be another device such as a desktop computer.

In the following embodiments, optional features and examples are provided simultaneously in each embodiment, and features recorded in the embodiments can be combined to form a plurality of optional solutions. Each numbered embodiment should not be considered as only one technical solution.

In one embodiment, as shown in FIG. 2, a prenatal screening information processing method is provided. An example in which the method is applied to the control terminal 110 in FIG. 1 is used for explanation. The following steps are included:
Step 101. Display a prenatal screening information processing interface.

The prenatal screening information processing interface is an interface for displaying and modifying pregnant woman information in a prenatal screening report system client installed on the terminal 110. Content rendered and displayed on the prenatal screening information processing interface is determined based on pregnant woman information selected by a user from the prenatal screening report system client.

The pregnant woman information is a set of information associated with a pregnant woman after the pregnant woman is registered. The pregnant woman information generally includes basic information of the pregnant woman, test information obtained after a submitted sample of the pregnant woman is tested, and fetus information of a fetus associated with the pregnant woman. Further, the fetus information specifically includes essential information of the fetus, risk information of the fetus, and the like. In some application scenarios, the pregnant woman information further includes diagnostic recommendations.

The basic information in the pregnant woman information is generally some basic information that needs to be entered by the pregnant woman needs to enter after the pregnant woman is registered and establishes a file in a hospital, and includes a sample number. The sample number is unique identification information of the pregnant woman in a database of a hospital information management system such as the laboratory information management system 120 or the prenatal screening risk calculation software. The only pregnant woman is determined based on the unique identification information. The basic information further includes medical record number, name, ID number, age, weight, ethnicity, history of diabetes, smoke, history of Trisomy 21 pregnancy, and the like, and further includes a multifetal pregnancy identification of the pregnant woman, to identify whether the pregnant woman carries a plurality of fetuses.

Further, if the pregnant woman had relevant examination data before, the basic information may further include a sample barcode, historical examination information, or the like. The sample barcode is a unique barcode of the submitted sample of the pregnant woman. The barcode is associated and bound with the sample number of the pregnant woman to associate test information of the corresponding sample with the corresponding pregnant woman.

In one embodiment, when a user selects data of a pregnant woman, the prenatal screening report system client parses relevant data in a background, renders corresponding display content based on the selected data and a user interface (UI) layout rule of a processing interface, and displays the display content on the prenatal screening information processing interface.

In another embodiment, after a user obtains the pregnant woman information from the laboratory information management system 120, such as a laboratory information system (LIS), based on the prenatal screening report system server 140 through the prenatal screening report system client, or after a user directly obtains the pregnant woman information from the prenatal screening risk calculation software through the prenatal screening report system client, the prenatal screening report system client displays the obtained pregnant woman information in a corresponding region of the prenatal screening information processing interface based on the obtained pregnant woman information.

In still another embodiment, after a user obtains the pregnant woman information from an LIS through the prenatal screening report system client based on the prenatal screening report system server 140 or directly obtains the pregnant woman information from the prenatal screening risk calculation software through the prenatal screening report system client, one of a plurality of pregnant women is selected as a default display pregnant woman, and the pregnant woman information of the default display pregnant woman is displayed in a corresponding region of the prenatal screening information processing interface. Usually, a pregnant woman in the first place in the pregnant woman information is selected as the default display pregnant woman.

The following exemplifies an application scenario in which the prenatal screening report system client directly obtains the pregnant woman information from the prenatal screening risk calculation software in the above embodiment: After a user first builds the prenatal screening report system server 140 in the hospital information management system and installs the prenatal screening report system client on the control terminal 110, in order to directly employ the prenatal screening report system client to view and review the pregnant woman information in subsequent operations, originally used pregnant woman information stored in the prenatal screening risk calculation software needs to be introduced into the prenatal screening report system client, and then the pregnant woman information is transmitted to the prenatal screening report system server 140 through the prenatal screening report system client for storage and backup of the pregnant woman information on the prenatal screening report system server 140.

Step 102. If a target pregnant woman is in a multifetal pregnancy state, display a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

Multifetal pregnancy means that the target pregnant woman carries two or more fetuses. Each fetus has corresponding essential information such as an NT value, presence or absence of nasal bone, a Crown-rump length (CRL) value, gestational week information such as an ultrasound gestational week or a sampling gestational week, and risk information such as an MOM value, a maternal age risk, and a trisomy syndrome risk, etc.

Each first information component is configured to display fetus information in the pregnant women information. The first information component includes a plurality of display units classified based on different types of the displayed fetus information, such as an essential information unit for displaying essential information of a fetus and a risk information unit for displaying risk information of the fetus.

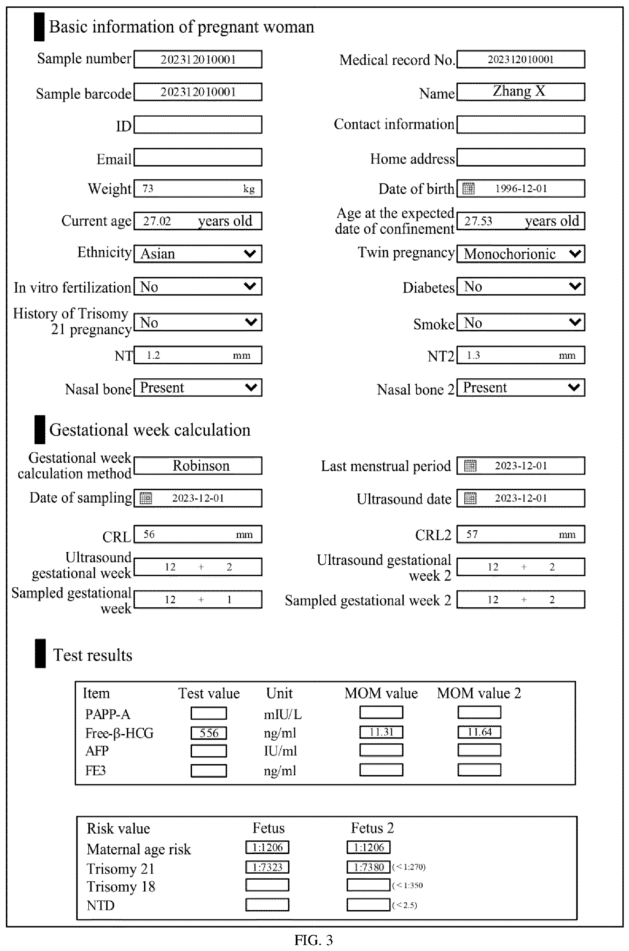

The units, as shown in FIG. 3, may be classified based on types of displayed information and displayed on the prenatal screening information processing interface together. For example, the NT information, the gestational week information, the MOM values, and the like of two fetuses are displayed side by side in the same row or column within a region. Further, some fetus information may be displayed in a display region for the basic information of the pregnant woman, such as the NT information and presence or absence of the nasal bone, or may be displayed in a display region for the test information of the pregnant woman, as shown in a test result region in FIG. 3, such as the MOM values. The essential information unit and the risk information unit are artificially classified based on a type of display content. On the prenatal screening information processing interface, the units do not limit display regions. For example, if the NT, presence or absence of the nasal bone, the CRL information, and other content are displayed, one or more pieces of content may be displayed intensively in the same display region or separately displayed in different display regions.

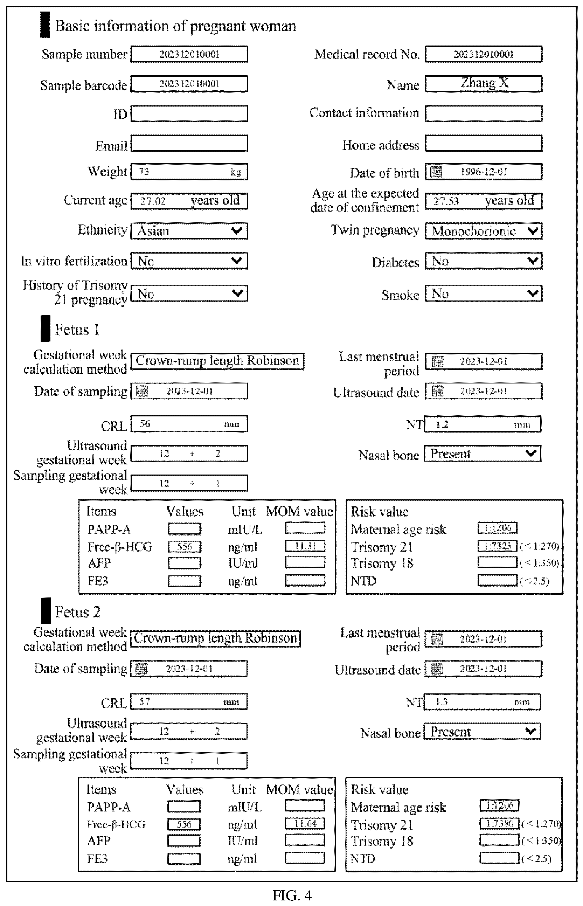

Further, as shown in FIG. 4, classification may be performed based on fetuses. On fetus basis, fetus information of fetuses is displayed intensively on a prenatal screening information processing interface. Essential information and risk information of fetus 1 are displayed intensively in a display region for fetus 1, and essential information and risk information of fetus 2 are displayed intensively in a display region for fetus 2. In addition, test information of a pregnant woman may be further repeatedly displayed in the display regions of the fetuses, to correspond to the MOM values in the risk information.

According to the prenatal screening information processing method, prenatal screening information is displayed on the prenatal screening information processing interface. When a selected pregnant woman carries a plurality of fetuses, information of the plurality of fetuses is intensively and synchronously displayed on the prenatal screening information processing interface, making it convenient for a user to intensively view the information. Since one pregnant woman has already carried information of the plurality of fetuses, the user does not need to enter information of other fetuses after creating a pregnant woman file, thereby avoiding repeated entry of basic information of pregnant women, simplifying entry steps, and improving reviewing efficiency.

In one embodiment, the prenatal screening information processing interface further includes a second information component; the second information component includes a basic information unit and a test information unit; and the method further includes: displaying basic information of the target pregnant woman by the basic information unit, displaying test information of the target pregnant woman by the test information unit, and displaying the essential information of the corresponding fetuses through the essential information units, where the basic information includes a multifetal pregnancy identification; the multifetal pregnancy identification is used to identify whether the target pregnant woman is in the multifetal pregnancy state; and determining the risk information of each fetus based on at least one of the basic information of the target pregnant woman, the test information of the target pregnant woman, and the basic information of each fetus.

The second information component includes a basic information display unit and a test information display unit. The basic information display unit is configured to display the basic information of the pregnant woman, and the test information display unit is configured to display the test information obtained based on a submitted sample of the pregnant woman. Test values of test items such as PAPPA, free-β-HCG, AFP, and FE3 in the test information are directly obtained by a test instrument based on the submitted sample of the pregnant woman. The test instrument is usually a sample analyzer. As shown in FIG. 3, in order to facilitate user query, the basic information and test information of the pregnant woman are usually intensively displayed in a designated region of the prenatal screening information processing interface.

A user selects a designated pregnant woman as the target pregnant woman through the prenatal screening report system client, and the pregnant woman information of the target pregnant woman is displayed in a relevant region. When the multiple pregnancy identification in the basic information of the pregnant woman indicates that the pregnant woman is a multifetal pregnant woman, the prenatal screening report system client further respectively displays, based on the fetus information of each fetus, the essential information and the risk information that correspond to the fetuses on the prenatal screening information processing interface through the display units, namely the essential information unit and the risk information unit. The MOM value in the risk information is related to a test result of each test item in the test information and is specifically a ratio of a test value of the corresponding item of the submitted sample of the pregnant woman to a median of test values of the item of a normal pregnant group at this gestational week. If the pregnant woman is in the multifetal pregnancy state, gestational weeks of the fetuses may be different. Therefore, a corresponding MOM value needs to be calculated for each fetus. That is, the risk information is calculated comprehensively based on the basic information of the pregnant woman with reference to the essential information of the fetus and the test information of the sample. Further, risk values of the items in the risk information also affect calculation of other risk items. For example, the MOM value affects the calculation of risk of trisomy 21 and the calculation of risk of trisomy 18. The history of trisomy 21 pregnancy may affect the calculation of maternal age risk, which then affects the calculation of risk of trisomy 21. The result about the nasal bone directly affects the calculation of risk of trisomy 21. Specifically, an example in which sub-risk values of some risk items are calculated is used.
Risk of Trisomy 21:
   1) Single marker test in first trimester: NT;
   2) Double marker test in first trimester (+NT): free-β-HCG+PAPP-A (+NT);
   3) Single marker test in second trimester: AFP;
   4) Double marker test in second trimester (+NT): AFP+free-β-HCG (+NT);
   5) Triple marker test in second trimester (+NT): AFP+free-β-HCG+FE3 (+NT);
Risk of Trisomy 18:
   1) Double marker test in first trimester (+NT): free-β-HCG+PAPP-A (+NT);
   2) Double marker test in second trimester (+NT): AFP+free-β-HCG (+NT);
   3) Triple marker test in second trimester (+NT): AFP+free-β-HCG+FE3 (+NT), where:
      1. The single marker test means using one type of marker or NT to calculate a risk. The double marker test means using two types of markers to calculate a risk. The triple marker test means using three types of markers to calculate a risk. 2. The double marker test (+NT) means that two types of markers may be used to calculate a risk or two types of markers + NT may be used to calculate a risk. The triple marker test (+ NT) is conducted in the similar way.

In one embodiment, the displaying basic information of the target pregnant woman by the basic information unit, displaying test information of the target pregnant woman by the test information unit, and displaying the essential information of the corresponding fetuses through the essential information units includes: in response to a first entering operation triggered on the second information component, respectively displaying, in the basic information unit and the test information unit, the basic information and test information of the target pregnant woman that are entered based on the first entering operation; and in response to a second entering operation triggered on the first information components, displaying, in the corresponding essential information units, the essential information of each fetus entered based on the second entering operation.

The first entering operation is an information entering operation that is performed by a user on the second information component on the prenatal screening information processing interface, to manually enter the pregnant woman information such as the basic information and the test information into the prenatal screening report system client for displaying, archiving in a local terminal, backup in the prenatal screening report system server, or the like.

The second entering operation is an information entering operation that is performed by a user on the first information components on the prenatal screening information processing interface, to manually enter the essential information and the like of each fetus into the prenatal screening report system client for displaying, archiving in a local terminal, backup in the prenatal screening report system server, or the like.

In an application scenario, the control terminal 110 and the prenatal screening report system server 140 are used offline separately, without communicative connection to other systems, the test instrument, or the prenatal screening risk calculation software. In this case, the control terminal 110 cannot obtain data stored in other systems, the test instrument, or the software through communication means. When the prenatal screening report system server 140 has pre-stored pregnant woman information, a user can directly obtain and display the pregnant woman information through the prenatal screening information processing interface of the prenatal screening report system client. If the data database of the prenatal screening report system server 140 stores no pregnant woman information, the user can also manually create a pregnant woman file through the prenatal screening information processing interface and enter relevant pregnant woman information. Meanwhile, the second information component displays the basic information of the pregnant women that is manually entered by the user in the designated region, such as the sample number, the name of the pregnant woman, the date of birth, and the ethnicity. Similarly, the user can respectively enter content corresponding to the information through display controls of the information in the units such as the essential information unit in the first information component. When the information required for the various risk calculations described above is entered, the risk information unit automatically displays calculation results of various risk values, and the user does not need to manually control triggering of a risk calculation function.

In another application scenario, when the control terminal 110 and the prenatal screening report system server 140 establish a communicative connection to the laboratory information management system 120, if the database of the prenatal screening report system server 140 or the database of the laboratory information management system 120 stores no pregnant woman information, a user can also manually create a pregnant woman file through the prenatal screening information processing interface and enter relevant pregnant woman information. Meanwhile, the second information component displays the basic information of the pregnant women that is manually entered by the user in the designated region, such as the sample number, the name of the pregnant woman, the date of birth, and the ethnicity. Similarly, the user can respectively enter content corresponding to the information through display controls of the information in the units such as the essential information unit in the first information component. When the information required for the various risk calculations described above is entered, the risk information unit automatically displays calculation results of various risk values, and the user does not need to manually control triggering of a risk calculation function.

In one embodiment, the displaying basic information of the target pregnant woman by the basic information unit, displaying test information of the target pregnant woman by the test information unit, and displaying the essential information of the corresponding fetuses through the essential information units further includes: obtaining, from an associated information management system, the basic information and test information of the target pregnant woman, and the essential information of each fetus of the target pregnant woman; and respectively displaying the basic information and test information of the target pregnant woman in the basic information unit and the test information unit, and displaying the essential information of the corresponding fetuses in the essential information units.

The associated information management system is an information management system that establishes a communicative connection to the prenatal screening report system server 140. In the hospital information management system, the information management system usually includes an outpatient system of a hospital, a laboratory information management system, a comprehensive information management system for test instruments, and the like. The prenatal screening report system server 140 transmits an application programming interface (API) opening request to the associated information management system. After receiving the request, the system parses content of the request and opens a corresponding interface for the prenatal screening report system server 140 to access. The prenatal screening report system server 140 obtains corresponding pregnant woman information and fetus information included in the pregnant woman information. The prenatal screening report system server 140 also obtains relevant test information when the above information includes the relevant test information. The obtained pregnant woman information is usually presented in the form of a list on the prenatal screening information processing interface of the prenatal screening report system client. After a user selects a pregnant woman, i.e. the target pregnant woman, who is expected to be reviewed, the software calls the cached pregnant woman information from the local control terminal 110 or retrieves the stored pregnant woman information from the database of the prenatal screening report system server 140, and displays the basic information of the selected pregnant woman, the corresponding test information (if any), the essential information (if any) of each fetus, and the risk information (if any) of each fetus on the prenatal screening information processing interface.

In one embodiment, the associated information management system includes a laboratory information management system; the prenatal screening information processing interface includes a data processing region; the data processing region includes a review state control; the obtaining, from an associated information management system, the basic information and test information of the target pregnant woman, and the essential information of each fetus of the target pregnant woman includes: in response to a triggering operation on the review state control, transmitting an update request to the laboratory information management system, where the update request is used to instruct the laboratory information management system to obtain unreviewed pregnant woman information from a database, and the pregnant woman information includes basic information and test information of a pregnant woman, and essential information of a corresponding fetus of the pregnant woman; receiving updated pregnant woman information transmitted by the laboratory information management system, and displaying a candidate pregnant women list in the data processing region based on the updated pregnant woman information; and determining the target pregnant woman from the candidate pregnant women list, and obtaining the basic information and test information of the target pregnant woman, and the essential information of each fetus of the target pregnant woman.

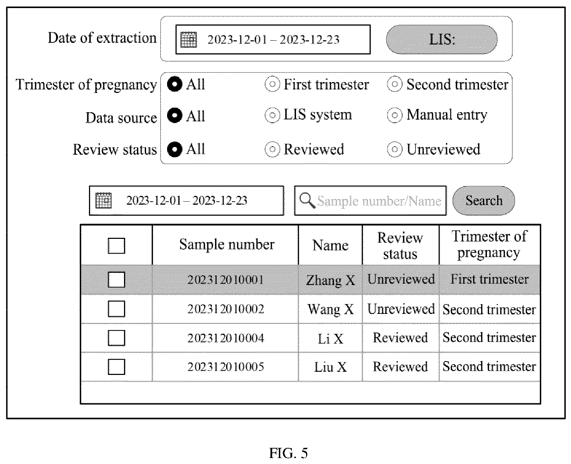

The review state control is an operation control for screening pregnant woman information that needs to be obtained on the prenatal screening information processing interface of the prenatal screening report system client. As shown in FIG. 5, a user selects Unreviewed on the review state control as a review state of pregnant woman information desired to be obtained, and the terminal generates a corresponding request instruction based on an input of the user and transmits the request instruction to the laboratory information management system. The system transmits, based on the request content, pregnant woman information that is stored in the database and is in an unviewed state to the terminal. The terminal receives returned data and sets a highest storage priority for the returned data. When there is a duplicate between the preceding pregnant woman information locally stored on the terminal and the returned pregnant woman information, the returned pregnant woman information is preferentially stored to overlay the preceding pregnant woman information to update information. The prenatal screening report system client parses and organizes the pregnant woman information in the unreviewed state based on the updated information, and displays the pregnant woman information in a candidate list. As shown in FIG. 5, the user can select a candidate pregnant woman from the candidate list one by one or once according to a review need. After the candidate pregnant woman is determined as the target pregnant woman, the pregnant woman information of the pregnant woman is presented on the prenatal screening information processing interface. After completing the review of the pregnant woman information, the user can manually modify the review state of the pregnant woman information review state to Reviewed on an operation interface and return the updated information to the laboratory information management system 120 to complete bidirectional synchronization of data.

In another embodiment, the review state control further includes other screening factors such as a trimester of pregnancy (first trimester and second trimester), and a data source (an LIS system and manual entry). The LIS system is the laboratory information management system. By determining the above screening factors and combining the review state, a user can more accurately obtain desired pregnant woman information, thereby reducing data transmission for two-party interaction and reducing a workload of screening by the user.

In a general application scenario, the control terminal 110 usually needs to communicate and interact with the hospital information management system, especially the laboratory information management system 120. A current mainstream method is that the control terminal 110 installed with the prenatal screening report system client communicates continuously with the laboratory information management system 120 through the prenatal screening report system server 140 to ensure timeliness and accuracy of the pregnant woman information. However, frequent transmission of service requests to the laboratory information management system 120 will further increase a server load and affect system stability. If pregnant woman information obtained based on a period, unreviewed samples may be modified at any time. If sample information in the laboratory information management system 120 changes after last extraction, the sample information in the prenatal screening report system client is still the same as that in the last extraction. If the updated pregnant woman information is reviewed and backhauled, the laboratory information management system 120 may store outdated and incorrect pregnant woman information, leading to a significant deviation between a subsequent diagnostic result and an actual situation, which may easily cause medical accidents. Therefore, the review state control with a function of screening review states of pregnant woman information is added. By selecting corresponding unreviewed pregnant woman information, the control terminal 110 can extract the pregnant woman information again and refresh pregnant woman information in the prenatal screening report system client, to ensure that information in the prenatal screening report system client is up-to-date. Meanwhile, modifying a review state of reviewed pregnant woman information and backhauling the state to the laboratory information management system 120 ensures consistency and accuracy of data storage at two parties. In addition, obtaining information on a designated pregnant woman according to real-time clinical needs also conforms to operation habits of medical staff.

In one embodiment, the determining the risk information of each fetus based on at least one of the basic information of the target pregnant woman, the test information of the target pregnant woman, and the basic information of each fetus further includes: synchronizing fetus information of each fetus corresponding to the target pregnant woman to prenatal screening risk calculation software for storage, the fetus information includes at least one of the essential information and the risk information.

The prenatal screening risk calculation software is a professional software with a plurality of fetus risk value calculation algorithms. It is usually installed on the control terminal 110 for calling by a user. However, since the prenatal screening risk calculation software only supports data storage of one pregnant woman associated with one fetus, in this embodiment, the prenatal screening risk calculation software only provides an algorithm support for risk value calculation and MOM value calculation, and view and review of pregnant woman information are completed at the prenatal screening report system client installed on the control terminal 110. When calculating the risk information of a fetus, the prenatal screening report system client transmits relevant data to the prenatal screening risk calculation software. The prenatal screening risk calculation software calls a corresponding algorithm to calculate and obtain a result of a risk value or an MOM value, then returns the result to the prenatal screening report system client, and displays the result in a corresponding region of the prenatal screening information processing interface.

Further, the prenatal screening report system client may further synchronize the pregnant woman information including the fetus information to the prenatal screening risk calculation software to meet a compliance requirement of the medical industry for pregnant woman information storage and also ensure consistency of data storage between a plurality of software. Similarly, the prenatal screening risk calculation software may further synchronize the pregnant woman information including the fetus information to the prenatal screening report system client. In a case that the prenatal screening report system client and the prenatal screening risk calculation software support mutual synchronous of data information, when the prenatal screening risk calculation software introduces data to the prenatal screening report system client, mismatching or inconsistent of data can be avoided.

In one embodiment, the synchronizing fetus information of each fetus corresponding to the target pregnant woman to prenatal screening risk calculation software for storage includes: determining a first fetus and at least one second fetus from a plurality of fetuses based on at least one of the essential information and the risk information; calling a synchronization interface of the prenatal screening risk calculation software, and synchronizing a first data file to a first storage position of the prenatal screening risk calculation software, where the first data file includes the basic information and test information of the target pregnant woman, and the fetus information of the first fetus; the first storage position is used to store data that is recognized and displayed by the prenatal screening risk calculation software; and synchronizing a second data file to a second storage position of the prenatal screening risk calculation software, where the second data file includes the basic information and test information of the target pregnant woman, and the fetus information of the second fetus; and the second storage position is used to store data that does not need to be displayed by the prenatal screening risk calculation software.

In the synchronization between the prenatal screening report system client and the prenatal screening risk calculation software, one fetus carried by a multifetal pregnant woman may be determined as a default associated fetus based on a preset rule. The preset rule may be that a fetus with a largest risk value is bound to the pregnant woman as the default associated fetus based on the risk information of the fetus, or a fetus with a largest gestational week value calculated based on the basic information of the pregnant woman and the CRL or other parameters in the fetus information is bound to the pregnant woman as the default associated fetus, or a fetus with a lowest or highest risk is comprehensively evaluated with reference to the basic information of the pregnant woman and a plurality of pieces of risk information of the fetus and is bound to the pregnant woman as the default associated fetus.

After the default associated fetus of the pregnant woman is determined, data such as the pregnant woman information including the information of the default associated fetus is packaged, and a data interface of the prenatal screening risk calculation software is called to synchronize the data to the database in the prenatal screening risk calculation software. Data stored in the database is a basis for normal operation and displaying of functions of the prenatal screening risk calculation software, and its data storage format includes tables, records, fields, indexes, or the like. The prenatal screening risk calculation software associates the default associated fetus with the target pregnant women based on a storage logic in the database for calling and displaying on an operation interface of the prenatal screening risk calculation software by a user. For other fetuses of the pregnant woman, the prenatal screening report system client calls the data interface of the prenatal screening risk calculation software after packaging data including the pregnant woman information and information of each fetus such as the fetus information, the risk information, the test information, and a storage path of the data packet is specified to other storage positions except the database in the prenatal screening risk calculation software, such as a non database storage region in a local file and a backup region of a cloud server. Data storage of the above region is different from the storage logic in the database. The prenatal screening risk calculation software does not run or display data, and the prenatal screening risk calculation software does not need to read data and display data on the operation interface. Therefore, it is not necessary to verify whether the data stored by the prenatal screening risk calculation software has a duplication in the database or other storage regions, and the information of other fetuses and the information of the pregnant woman can be saved in the prenatal screening risk calculation software. Further, since the pregnant woman information stored in the database and the pregnant woman information in a data packet in other storage paths are the same, the pregnant woman information and the data of the associated fetus can be jointly synchronized to the prenatal screening report system client through the same unique pregnant woman identification when the prenatal screening risk calculation software backhauls the synchronized pregnant woman information to the prenatal screening report system client, to ensure integrity of data.

In another embodiment, the prenatal screening report system client packages the information of the fetuses carried by the same pregnant woman and the information of the pregnant woman, and then calls the data interface of the prenatal screening risk calculation software to transmit the information to the prenatal screening risk calculation software. The prenatal screening report system client does not specify storage paths of data packets in the prenatal screening risk calculation software before transmission. By performing different data identifications on the data packets, the prenatal screening risk calculation software determines a fetus with a highest risk value as the default associated fetus based on a preset rule, such as based on the risk information of the fetus, or may determine a fetus with a largest gestational week value that is calculated based on the basic information of the pregnant woman with reference to the information of the fetus such as the CRL or other parameters as the default associated fetus, or may comprehensively evaluate a fetus with a lowest or highest risk with reference to the basic information of the pregnant woman and a plurality of pieces of risk information of the fetus and determine the fetus as the default associated fetus. The data packets of the default associated fetus are put into the database for storage and are identified as one piece of pregnant woman and fetus information for displaying. The data packets of other fetuses and the pregnant woman are put into other storage regions or paths for data storage. When the data of the prenatal screening risk calculation software needs to be synchronized to the prenatal screening report system client, the data and the data of the associated fetus may be jointly synchronized to the prenatal screening report system client through the unique pregnant woman identification in the data packets of the different fetuses of the same pregnant woman, thereby ensuring integrity of data.

Since the prenatal screening risk calculation software only supports data storage for one pregnant woman and one associated fetus, when a pregnant woman carries a plurality of fetuses, the prenatal screening report system client may only save data of one fetus during the synchronization with the prenatal screening risk calculation software, which may lead to easy loss of data of other fetuses. By using the above method, while the data of all the fetuses of the pregnant woman are successfully saved, the native storage logic of the prenatal screening risk calculation software is also satisfied. Since the data of other fetuses is redundant data in the storage logic of the prenatal screening risk calculation software, the data will not be displayed on the interface of the prenatal screening risk calculation software and there is no risk of misoperations such as deletion or modification. When the pregnant woman information needs to be synchronized to the prenatal screening report system client, data files of other fetuses are associated with the pregnant woman through the pregnant woman identification and synchronized to the prenatal screening report system client, thereby ensuring consistency of data synchronization between two software and also meeting a compliance requirement of the medical industry.

In one embodiment, the essential information unit includes a gestational week calculation method control; the essential information further includes a gestational week calculation result; and a mode for obtaining the gestational week calculation result includes: in response to a triggering operation on the gestational week calculation method control, determining a gestational week calculation method; displaying a corresponding gestational week calculation factor entering unit based on the gestational week calculation method; and when an entering operation on the gestational week calculation factor entering unit satisfies a preset calculation condition, displaying the gestational week calculation result, where the preset calculation condition includes a value entered by the entering operation being within a preset calculation range.

The essential information unit further includes the gestational week calculation method control for allowing a user to select a gestational week calculation method of a fetus. As shown in FIG. 3 or FIG. 4, the gestational week calculation method control currently selects a calculation method of "Crown-rump length Robinson", and the corresponding gestational week calculation factor entering unit provides entering units for factors such as date of sampling, ultrasound date, and CRL for entering by the user. When the user sequentially enters a date of sampling, an ultrasound date, and a CRL value of the pregnant woman, whether the above values are within preset calculation ranges is determined one by one. For example, when a preset CRL calculation range is 4 to 100 mm and the CRL value entered by the user is 3, the ultrasound gestational week of the fetus and sampling gestational week will not be automatically calculated. When the CRL value of fetus 1 entered by the user is 56, and the CRL value of fetus 2 entered by the user is 57, calculation results will be automatically displayed in ultrasound gestational week and sampling gestational week display regions corresponding to the fetuses. The gestational week calculation methods and the corresponding gestational week calculation factors that need to be entered are as follows:

| | Gestational week calculation method | Gestational week calculation factor |
|---|---|---|
| 1 | Crown-rump length Robinsion | Date of sampling, ultrasound date, CRL |
| 2 | Crown-rump length Own Table | Date of sampling, ultrasound date, CRL, ultrasound gestational week |
| 3 | Biparietal diameter Hadlock | Date of sampling, ultrasound date, BPD |
| 4 | Biparietal diameter Own Table | Date of sampling, ultrasound date, BPD, ultrasound gestational week |
| 5 | Date of sampling | Date of sampling, sampling gestational week |
| 6 | Expected date of confinement | Date of sampling, expected date of confinement |
| 7 | Last menstrual period (LMP) | Last menstrual period, date of sampling, menstrual cycle |
| 8 | IVF | Date of sampling, sampling gestational week |

By determining whether the values entered by the user meet preset conditions, invalid content entered by the user can be effectively filtered out, and the user can be further reminded of errors in the entering operation. This lowers an operation threshold and improves fault tolerance of a system, thereby further avoiding a wrong diagnostic result provided in subsequent diagnostic assessment caused by a deviation between data stored for pregnant women and actual data due to a data entry error, avoiding medical accidents, and improving stability of use of a system. Meanwhile, the gestational week calculation method control can provide various gestational week calculation methods for selection by the user, so that the user can link and display different risk calculation results based on different gestational week calculation methods, which better helps the user in diagnosis.

In one embodiment, the prenatal screening information processing method further includes: in response to a triggering operation on the report generation control, generating a prenatal screening report, prenatal screening report includes the essential information and the risk information that respectively correspond to the plurality of fetuses of the target pregnant woman.

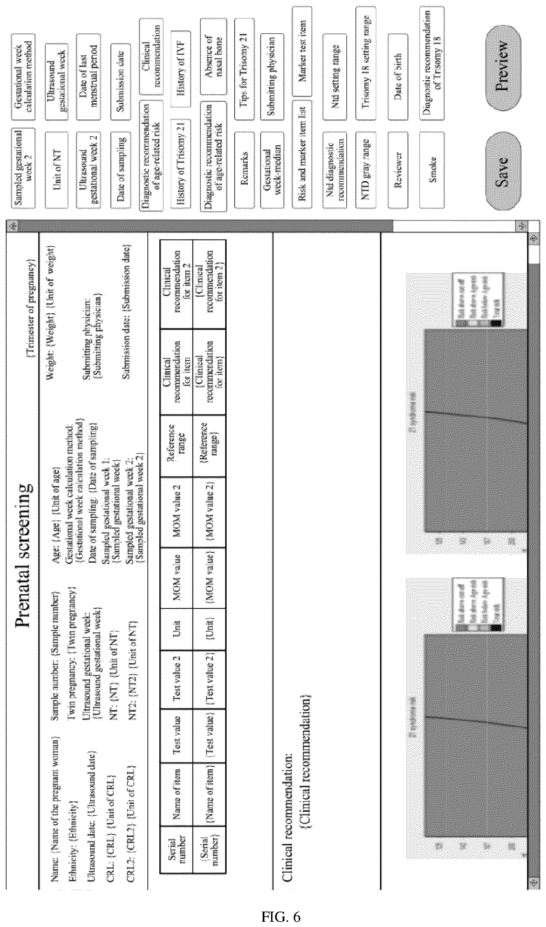

The prenatal screening information processing interface further includes the report generation control for outputting the selected pregnant woman information according to a report template. An output report may be sent out in the form of an electronic file through chat software, a USB flash disk copy, or an email, or may be printed as a paper report. The report output according to the report template includes the basic information of the pregnant woman and the information of the plurality of fetuses, such as the essential information and the risk information, and may further include other information such as risk diagnosis recommendations. Specifically, the essential information and risk information of a fetus needing to be presented may be manually adjusted by the user after the user clicks the report generation control on the prenatal screening information processing interface. As shown in FIG. 6, a user may customize information of a fetus expected to be displayed according to specific needs, such as NT information, a risk value for an item, and risk diagnosis recommendations. Further, display units of various types of information are modularized and may be placed arbitrarily in a report display region, thereby meeting different needs of different users for presentation content and a layout while ensuring that the information of the plurality of fetuses are displayed simultaneously in one report.

Due to limitations of the prenatal screening risk calculation software, one pregnant woman can only be associated with one fetus. When a prenatal screening report of a multifetal pregnant woman needs to be output or printed, the information of other fetuses cannot be displayed in one report. This has poor readability for patients and doctors. In addition, the user cannot quickly and accurately associate other fetuses with the pregnant woman, so that there is a risk of confusion between fetuses and pregnant women. Through the multifetal report template of this solution, the information of a plurality of fetuses can be displayed together in one report, which improves readability of users, ensures correlation and continuity between a plurality of fetuses and a pregnant woman, and eliminates a risk of report confusion.

In one embodiment, a user runs the prenatal screening report system client through the control terminal 110, and the prenatal screening report system client displays the prenatal screening information processing interface after being run. The user obtains complete pregnant woman information by transmitting an interface request to an LIS system, namely the laboratory information management system 120, and can obtain the test information of the submitted sample of the pregnant woman by transmitting an interface request to the test instrument 130. Further, the complete pregnant woman information may be obtained by obtaining data from the prenatal screening risk calculation software or extracting local data which is the data stored on the delivery screening report system server 140. Still further, the basic information of the pregnant woman, the test information of the pregnant woman, and the essential information of each fetus of the pregnant woman may also be obtained through manual entry or the like, and the risk information may be calculated based on the above information. In summary, the test instrument 130 only provides the test information of the submitted sample of the pregnant woman. The LIS system, the prenatal screening risk calculation software, the prenatal screening report system server 140, and the manual entry method may provide the basic information and test information of the pregnant woman and the essential information and risk information of the associated fetuses.

In the obtaining method implemented by transmitting the interface request to the LIS system, the user can transmit a relevant data obtaining request to the LIS system after determining screening parameters such as a date of extraction, a trimester of pregnancy, a data source, and a review state. After receiving returned relevant data, the user can further define a date of extraction in a candidate pregnant woman display column, to reduce a number of candidate pregnant women.

After the user obtains relevant pregnant woman information and determines the target pregnant woman expected to be viewed from the candidate pregnant woman display column, the associated basic information, the test information (if any), and the relevant information (if any) of the fetus are displayed on the prenatal screening information processing interface. In a scenario, the relevant information of the fetus lacks a CRL value. After the user selects "Crown-rump length Robinson" as the gestational week calculation method, filling controls of the date of sampling, the ultrasound date, the CRL, and other information are arranged in an order on the prenatal screening information processing interface. When data includes the date of sampling and the ultrasound date, corresponding filling controls automatically enter the values and waits for the user to enter the CRL value. When the CRL value entered by the user satisfies a set calculation range (4 to 100 mm), for example, when the CRL is 56 mm, a back-end automatically calculates a corresponding ultrasound gestational week and a corresponding sampling gestational week. In another scenario, when the entered pregnant woman information includes a plurality of pieces of information for calculating an ultrasound gestational week and a sampling gestational week, such as a date of sampling, an ultrasound date, and a CRL based on the "Crown-rump length Robinsion" calculation method, or a date of sampling, an ultrasound date, and a BPD based on the "biparietal diameter Hadlock" calculation method, corresponding filling controls are displayed while using one calculation method as a default calculation method.

Further, when the pregnant woman information further includes the test information of the pregnant woman, test results are displayed in corresponding test items based on a type of test information, and corresponding fetus risk values are calculated in a risk value column. Meanwhile, diagnostic results are output in a diagnostic recommendation column. The calculation of the risk values is not only based on the test results of the relevant items, but also on the essential information of the fetus and the basic information of the pregnant woman. For a specific relationship, refer to the section of calculating sub-risk values of some risk items in the previous text.

After completing the review of the pregnant woman, the user can save the information by clicking a save button. Meanwhile, the pregnant woman information is output or printed according to the report template. Display content of the report module is modularized. The user can customize the display content and display positions of the content.

Further, after the review and outputting of the information are completed, the user can select the corresponding pregnant woman information and synchronize the information to the prenatal screening risk calculation software and the LIS system, thereby completing data synchronization between systems.

It should be understood that although the steps are displayed sequentially according to the instructions of the arrows in the flowcharts of the embodiments, these steps are not necessarily performed sequentially according to the sequence instructed by the arrows. Unless otherwise explicitly specified in the present invention, execution of the operations is not strictly limited, and the operations may be performed in other sequences. Moreover, at least some of the steps in the flowcharts of the various embodiments may include a plurality of steps or a plurality of stages. These steps or stages are not necessarily performed at the same moment but may be performed at different moments. Execution of these steps or stages is not necessarily performed in sequence, but may be performed in turn or alternately with other steps or at least some of steps or stages of other steps.

Based on the same inventive concept, an embodiment of the present invention further provides a prenatal screening information processing apparatus for implementing the above prenatal screening information processing method. An implementation solution provided by the apparatus to solve the problems is similar to the implementation solution recorded in the above method. Therefore, for specific limitations on the one or more embodiments of the prenatal screening information processing apparatus provided below, refer to the limitations on the prenatal screening information processing method mentioned above, and details will not be elaborated here.

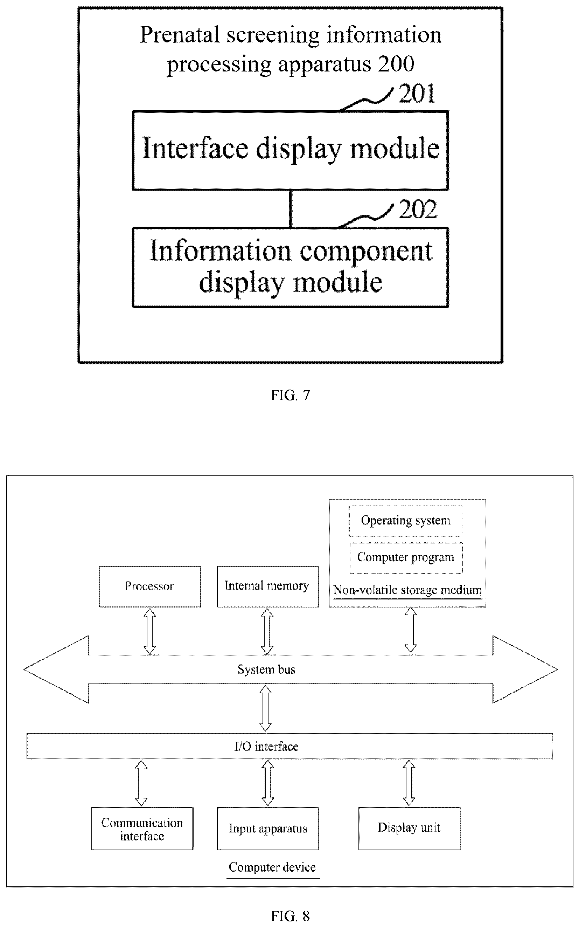

In one embodiment, as shown in FIG. 7, a prenatal screening information processing apparatus 200 is provided, including:
an interface display module 201, configured to display a prenatal screening information processing interface; and
an information component display module 202, configured to: if a target pregnant woman is in a multifetal pregnancy state, display a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

The modules in the prenatal screening information processing apparatus may be implemented entirely or partially through software, hardware, or a combination thereof. The foregoing modules may be built in or independent of a processor of a computer device in a form of hardware, or may be stored in a memory of the computer device in a form of software, for the processor to invoke to execute operations corresponding to the foregoing modules.

In one embodiment, a computer device is provided. The computer device may be a server, a diagram of an internal structure of which can be as shown in FIG. 8. The computer device includes a processor, a memory, a and a network interface which are connected through a system bus. The processor of the computer device is configured to provide computing and control capabilities. The memory of the computer device includes a non-volatile storage medium and an internal memory. The non-volatile storage medium stores an operating system, computer programs, and databases. The internal memory provides an environment for running the operating system and the computer program in the non-volatile storage medium. The database of the computer device is configured to store pregnant woman information including fetus information. The network interface of the computer device is used to communicate with an external terminal through network connection. The computer program is executed by the processor to implement a prenatal screening information processing method.

In one embodiment, a computer device is provided. The computer device may be a terminal, a diagram of an internal structure of which can be as shown in FIG. 8. The computer device includes a processor, a memory, a communication interface, a display screen, and an input device which are connected through a system bus. The processor of the computer device is configured to provide computing and control capabilities. The memory of the computer device includes a non-volatile storage medium and an internal memory. The non-volatile storage medium stores an operating system and a computer program. The internal memory provides an environment for running the operating system and the computer program in the non-volatile storage medium. The communication interface of the computer device is configured for conducting wired or wireless communication with an external terminal. The wireless communication may be implemented through wireless fidelity (WIFI), a mobile cellular network, near field communication (NFC), or another technology. The computer program is executed by the processor to implement a prenatal screening information processing method. The display screen of the computer device may be a liquid crystal display screen or an e-ink display screen. The input device of the computer device may be a touch layer covering the display screen, or may be a button, a trackball, or a touchpad arranged on a housing of the computer device, or may be an external keyboard, touchpad, a mouse or the like.

A person skilled in the art can understand that the structure shown in FIG. 8 is merely a block diagram of a partial structure related to a solution in the present invention, and does not constitute a limitation on the computer device to which the solution in the present invention is applied.

Specifically, the computer device may include more or fewer components than those shown in the figure, or some components may be combined, or a different component layout may be used.

In one embodiment, a computer device is provided, including a memory and a processor. The memory has a computer program stored therein. The processor, when executing the computer program, implements the following steps:
Step 101. Display a prenatal screening information processing interface.
Step 102. If a target pregnant woman is in a multifetal pregnancy state, display a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

In one embodiment, the processor, when executing the computer program, further implements the following steps:
displaying basic information of the target pregnant woman by the basic information unit, displaying test information of the target pregnant woman by the test information unit, and displaying the essential information of the corresponding fetuses through the essential information units, where the basic information includes a multifetal pregnancy identification; the multifetal pregnancy identification is used to identify whether the target pregnant woman is in the multifetal pregnancy state; and determining the risk information of each fetus based on at least one of the basic information of the target pregnant woman, the test information of the target pregnant woman, and the basic information of each fetus.

In one embodiment, the processor, when executing the computer program, further implements the following steps:
In response to a first entering operation triggered on the second information component, respectively displaying, in the basic information unit and the test information unit, the basic information and test information of the target pregnant woman that are entered based on the first entering operation; and in response to a second entering operation triggered on the first information components, displaying, in the corresponding essential information units, the essential information of each fetus entered based on the second entering operation.

In one embodiment, a computer-readable storage medium is provided, having a computer program stored therein. The computer program, when executed by a processor, implements the following steps:
Step 101. Display a prenatal screening information processing interface.
Step 102. If a target pregnant woman is in a multifetal pregnancy state, display a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

In one embodiment, the computer program, when executed by the processor, further implements the following steps:
displaying basic information of the target pregnant woman by the basic information unit, displaying test information of the target pregnant woman by the test information unit, and displaying the essential information of the corresponding fetuses through the essential information units, where the basic information includes a multifetal pregnancy identification; the multifetal pregnancy identification is used to identify whether the target pregnant woman is in the multifetal pregnancy state; and determining the risk information of each fetus based on at least one of the basic information of the target pregnant woman, the test information of the target pregnant woman, and the basic information of each fetus.

In one embodiment, the computer program, when executed by the processor, further implements the following steps:
in response to a first entering operation triggered on the second information component, respectively displaying, in the basic information unit and the test information unit, the basic information and test information of the target pregnant woman that are entered based on the first entering operation; and in response to a second entering operation triggered on the first information components, displaying, in the corresponding essential information units, the essential information of each fetus entered based on the second entering operation.

In one embodiment, a computer program product is provided, including a computer program. The computer program, when executed by a processor, implements the following steps:
Step 101. Display a prenatal screening information processing interface.
Step 102. If a target pregnant woman is in a multifetal pregnancy state, display a plurality of first information components respectively corresponding to a plurality of fetuses on the prenatal screening information processing interface, where each first information component includes an essential information unit and a risk information unit; the essential information unit is configured to display essential information of corresponding fetuses; and the risk information unit is configured to display risk information of the corresponding fetuses.

In one embodiment, the computer program, when executed by the processor, further implements the following steps:
displaying basic information of the target pregnant woman by the basic information unit, displaying test information of the target pregnant woman by the test information unit, and displaying the essential information of the corresponding fetuses through the essential information units, where the basic information includes a multifetal pregnancy identification; the multifetal pregnancy identification is used to identify whether the target pregnant woman is in the multifetal pregnancy state; and determining the risk information of each fetus based on at least one of the basic information of the target pregnant woman, the test information of the target pregnant woman, and the basic information of each fetus.

In one embodiment, the computer program, when executed by the processor, further implements the following steps:
in response to a first entering operation triggered on the second information component, respectively displaying, in the basic information unit and the test information unit, the basic information and test information of the target pregnant woman that are entered based on the first entering operation; and in response to a second entering operation triggered on the first information components, displaying, in the corresponding essential information units, the essential information of each fetus entered based on the second entering operation.

It should be noted that user information (including but not limited to user device information and user personal information) and data (including but not limited to data for analysis, stored data, displayed data, and the like) involved in the present invention are information and data authorized by a user or fully authorized by all parties.

A person of ordinary skill in the art may understand that all or some of the procedures of the method in the foregoing embodiments may be implemented by the computer-readable instructions that instruct relevant hardware. The computer program may be stored in a non-volatile computer-readable storage medium. When the computer program is executed, the procedures of the foregoing method embodiments may be implemented. Any reference to a memory, a database, or other media used in the embodiments provided in the present invention can include at least one of a non-volatile memory and a volatile memory. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, or the like. The volatile memory can include a random access memory (RAM), an external cache memory, or the like. As an illustration, not a limitation, the RAM may be in many forms, such as a static random access memory (SRAM) or a dynamic random access memory (DRAM). The database involved in the embodiments of the present invention may include at least one of a relational database and a non-relational database. The non-relational database may include a blockchainbased distributed database, or the like, but is not limited thereto. The processor involved in the embodiments provided in the present invention may be a general-purpose processor, a central processing unit, a graphics processing unit, a digital signal processor, a programmable logic device, a quantum computing-based data processing logic device, or the like, but is not limited thereto.

All the technical features of the above embodiments can be combined randomly. For the sake of brevity, all possible combinations of all the technical features in the above embodiments are not described. However, these technical features shall all be considered to fall within the scope of this specification as long as there is no contradiction in their combinations.

The foregoing embodiments merely express several implementations of the present invention. The descriptions thereof are relatively specific and detailed, but for a person of ordinary skill in the art, several transformations and improvements can be made without departing from the idea of the present invention.

## Claims

1. A prenatal screening information processing method, comprising:
displaying a prenatal screening information processing interface (S101); and
if a target pregnant woman is in a multifetal pregnancy state, displaying a plurality of first information components respectively corresponding to a plurality of fetuses on the prenatal screening information processing interface (S102), wherein each piece of the first information component comprises an essential information unit and a risk information unit; the essential information unit is configured to display essential information of corresponding fetuses; and the risk information unit is configured to display risk information of the corresponding fetuses.

2. The prenatal screening information processing method according to claim 1, wherein the prenatal screening information processing interface further comprises a second information component; the second information component comprises a basic information unit and a test information unit; and the method further comprises:
displaying basic information of the target pregnant woman by the basic information unit, displaying test information of the target pregnant woman by the test information unit, and displaying the essential information of the corresponding fetuses through the essential information units, wherein the basic information comprises a multifetal pregnancy identification; the multifetal pregnancy identification is used to identify whether the target pregnant woman is in the multifetal pregnancy state; and
determining the risk information of each fetus based on at least one of the basic information of the target pregnant woman, the test information of the target pregnant woman, and the basic information of each fetus.

3. The prenatal screening information processing method according to claim 2, wherein the displaying basic information of the target pregnant woman by the basic information unit, displaying test information of the target pregnant woman by the test information unit, and displaying the essential information of the corresponding fetuses through the essential information units comprises:
in response to a first entering operation triggered on the second information component,
respectively displaying, in the basic information unit and the test information unit, the basic information and test information of the target pregnant woman that are entered based on the first entering operation; and
in response to a second entering operation triggered on the first information components, displaying, in the corresponding essential information units, the essential information of each fetus entered based on the second entering operation.

4. The prenatal screening information processing method according to claim 2, wherein the displaying basic information of the target pregnant woman by the basic information unit, displaying test information of the target pregnant woman by the test information unit, and displaying the essential information of the corresponding fetuses through the essential information units further comprises:
obtaining, from an associated information management system, the basic information and test information of the target pregnant woman, and the essential information of each fetus of the target pregnant woman; and
respectively displaying the basic information and test information of the target pregnant woman in the basic information unit and the test information unit, and displaying the essential information of the corresponding fetuses in the essential information units.

5. The prenatal screening information processing method according to claim 4, wherein the associated information management system comprises a laboratory information management system; the prenatal screening information processing interface comprises a data processing region; the data processing region comprises a review state control;
the obtaining, from an associated information management system, the basic information and test information of the target pregnant woman, and the essential information of each fetus of the target pregnant woman comprises:
in response to a triggering operation on the review state control, transmitting an update request to the laboratory information management system, wherein the update request is used to instruct the laboratory information management system to obtain unreviewed pregnant woman information from a database, and the pregnant woman information comprises basic information and test information of a pregnant woman, and essential information of a corresponding fetus of the pregnant woman;
receiving updated pregnant woman information transmitted by the laboratory information management system, and displaying a candidate pregnant women list in the data processing region based on the updated pregnant woman information; and
determining the target pregnant woman from the candidate pregnant women list, and obtaining the basic information and test information of the target pregnant woman, and the essential information of each fetus of the target pregnant woman.

6. The prenatal screening information processing method according to claim 2, wherein the determining the risk information of each fetus based on at least one of the basic information of the target pregnant woman, the test information of the target pregnant woman, and the basic information of each fetus further comprises:
synchronizing fetus information of each fetus corresponding to the target pregnant woman to prenatal screening risk calculation software for storage, wherein the fetus information comprises at least one of the essential information and the risk information.

7. The prenatal screening information processing method according to claim 6, wherein the synchronizing fetus information of each fetus corresponding to the target pregnant woman to prenatal screening risk calculation software for storage comprises:
determining a first fetus and at least one second fetus from a plurality of fetuses based on at least one of the essential information and the risk information;
calling a synchronization interface of the prenatal screening risk calculation software, and synchronizing a first data file to a first storage position of the prenatal screening risk calculation software, wherein the first data file comprises the basic information and test information of the target pregnant woman, and the fetus information of the first fetus; the first storage position is used to store data that is recognized and displayed by the prenatal screening risk calculation software; and
synchronizing a second data file to a second storage position of the prenatal screening risk calculation software, wherein the second data file comprises the basic information and test information of the target pregnant woman, and the fetus information of the second fetus; and the second storage position is used to store data that does not need to be displayed by the prenatal screening risk calculation software.

8. The prenatal screening information processing method according to any one of claims 2 to 4, wherein the essential information unit comprises a gestational week calculation method control; the essential information further comprises a gestational week calculation result; and a mode for obtaining the gestational week calculation result comprises:
in response to a triggering operation on the gestational week calculation method control, determining a gestational week calculation method; displaying a corresponding gestational week calculation factor entering unit based on the gestational week calculation method; and
when an entering operation on the gestational week calculation factor entering unit satisfies a preset calculation condition, displaying the gestational week calculation result, wherein the preset calculation condition comprises a value entered by the entering operation being within a preset calculation range.

9. The prenatal screening information processing method according to any one of claims 1 to 7, wherein the prenatal screening information processing interface further comprises a report generation control; and the method further comprises:
in response to a triggering operation on the report generation control, generating a prenatal screening report, wherein prenatal screening report comprises the essential information and the risk information that respectively correspond to the plurality of fetuses of the target pregnant woman.

10. A prenatal screening information processing apparatus, comprising:
an interface display module (201), configured to display a prenatal screening information processing interface; and
an information component display module (202), configured to: if a target pregnant woman is in a multifetal pregnancy state, display a plurality of first information components respectively corresponding to a plurality of fetuses on the prenatal screening information processing interface, wherein each piece of the first information component comprises an essential information unit and a risk information unit; the essential information unit is configured to display essential information of corresponding fetuses; and the risk information unit is configured to display risk information of the corresponding fetuses.

11. A computer device, comprising a memory and a processor, wherein the memory has a computer program stored therein, and the processor, when executing the computer program, implements the steps of the method according to any one of claims 1 to 9.

12. A computer-readable storage medium, having a computer program stored therein, wherein the computer program, when executed by a processor, implements the steps of the method according to any one of claims 1 to 9.
